# EUROPÄISCHE PATENTSCHRIFT

(11) **EP 1 401 445 B1**
(45) Veröffentlichungstag und Bekanntmachung des Hinweises auf die Patenterteilung: **23.08.2006**
(21) Anmeldenummer: 02743165.9
(22) Anmeldetag: 08.06.2002
(51) Int. Cl.: A61K 31/46, C07D 451/10

(54) **KRISTALLINES ANTICHOLINERGIKUM, VERFAHREN ZU DESSEN HERSTELLUNG UND DESSEN VERWENDUNG ZUR HERSTELLUNG EINES ARZNEIMITTELS**
CRYSTALLINE ANTICHOLINERGIC, METHOD FOR ITS PRODUCTION, AND USE THEREOF IN THE PRODUCTION OF A DRUG
SUBSTANCE ANTICHOLINERGIQUE CRISTALLINE, SON PROCEDE DE PRODUCTION ET SON UTILISATION POUR LA PRODUCTION D'UN MEDICAMENT

(30) Priorität: 22.06.2001 DE 10129710; 08.04.2002 DE 10215436
(43) Veröffentlichungstag der Anmeldung: 31.03.2004
(73) Patentinhaber: Boehringer Ingelheim Pharma GmbH & Co.KG, 55216 Ingelheim am Rhein (DE)
(72) Erfinder: SIEGER, Peter, 88441 Mittelbiberach (DE); WERTHMANN, Ulrike, 88400 Biberach (DE)
(86) Internationale Anmeldenummer: PCT/EP2002/006291
(87) Internationale Veröffentlichungsnummer: WO 2003/000265

(56) Entgegenhaltungen:
- EP-A- 0 418 716
- WO-A-02/30928
- WO-A-02/051840

## Beschreibung

Die Erfindung betrifft ein kristallines (1α,2β,4β,5α,7β,7β-[(hydroxydi-2-thienylacetyl)oxy]-9,9-dimethyl-3-oxa-9-azoniatricydo[3.3.1.0^{2,4}]nonane-bromid in der wasserfreien Form, Verfahren zu dessen Herstellung, sowie dessen Verwendung zur Herstellung eines Arzneimittels, insbesondere zur Herstellung eines Arzneimittels mit anticholinerger Wirkung.

### Hintergrund der Erfindung

Die Verbindung (1α,2β,4β,5α,7β)-7-[(hydroxydi-2-thienylacetyl)oxy]-9,9-dimethyl-3-oxa-9-azoniatricyclo[3.3.1.0^{2,4}]nonane-bromid, ist aus der Europäischen Patentanmeldung EP 418 716 A1 bekannt und weist die folgende chemische Struktur auf:

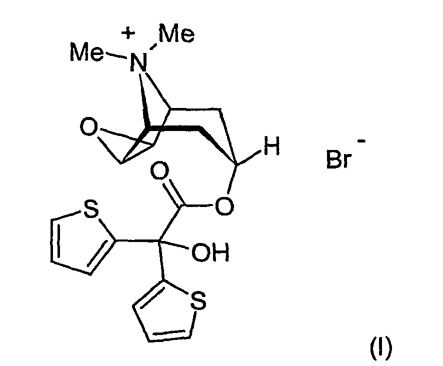

Die Verbindung besitzt wertvolle pharmakologische Eigenschaften und ist unter dem Namen Tiotropiumbromid (BA679BR) bekannt. Tiotropiumbromid stellt ein hochwirksames Anticholinergikum dar und kann deshalb bei der Therapie von Asthma oder COPD (chronic obstructive pulmonary disease = chronisch obstruktive Lungenerkrankung) einen therapeutischen Nutzen entfalten.

Die Applikation von Tiotropiumbromid erfolgt vorzugsweise auf inhalativem Wege. Hierbei können geeignete Inhaltionspulver, die in geeignete Kapseln (Inhaletten) abgefüllt mittels entsprechender Pulverinhalatoren appliziert werden, zum Einsatz kommen. Alternativ dazu kann eine inhalative Anwendung auch durch Applikation geeigneter Inhalationsaerosole erfolgen. Hierzu zählen auch pulverförmige Inhalationsaerosole, die beispielsweise HFA134a, HFA227 oder deren Gemisch als Treibgas enthalten.

Die richtige Herstellung der vorgenannten, zur inhalativen Verabreichung eines Arzneiwirkstoffes verwendbaren Zusammensetzungen stützt sich auf verschiedene Parameter, die mit der Beschaffenheit des Arzneiwirkstoffes selbst verbunden sind. Bei Arzneimitteln, die wie Tiotropiumbromid in Form von Inhalationspulvern oder Inhalationsaerosolen zur Anwendung gelangen, wird der kristalline Wirkstoff in gemahlener (mikronisierter) Form zur Herstellung der Formulierung verwendet. Da für die pharmazeutische Qualität einer Arzneimittelformulierung stets dieselbe kristalline Modifikation des Wirkstoffs gewährleistet sein muß, sind vor diesem Hintergrund an die Stabilität und Eigenschaften des kristallinen Wirkstoffes erhöhte Anforderungen zu stellen. Es ist besonders wünschenswert, den Wirkstoff in Form einer einheitlichen und klar definierten Kristallmodifikation bereitzustellen. Es ist ferner besonders wünschenswert, den Wirkstoff in einer kristallinen Form bereitzustellen, die nicht zur Bildung von Polymorphen neigt.

Neben den vorstehend angegebenen Erfordernissen ist generell zu berücksichtigen, dass jede Änderung des Feststoffzustandes eines Arzneimittels, welche dessen physikalische und chemische Stabilität verbessern kann, gegenüber weniger stabilen Formen desselben Arzneimittels einen erheblichen Vorteil ergibt.

Die Aufgabe der Erfindung besteht somit in der Bereitstellung einer neuen, stabilen Kristallform der Verbindung Tiotropiumbromid, die den vorstehend genannten hohen Anforderungen, die an einen Arzneimittelwirkstoff zu richten sind, genügen.

### Detaillierte Beschreibung der Erfindung

Es wurde gefunden, daß Tiotropiumbromid je nach Wahl der Bedingungen, die bei der Reinigung des nach der technischen Herstellung erhaltenen Rohprodukts angewendet werden können, in unterschiedlichen kristallinen Modifikationen anfällt.

Es wurde gefunden, daß diese unterschiedlichen Modifikationen maßgeblich durch Wahl der zur Kristallisation eingesetzten Lösemittel sowie durch Wahl der beim Kristallisationsprozess gewählten Verfahrensbedingungen gezielt erhalten werden können.

Es wurde überraschenderweise festgestellt, daß ausgehend vom Monohydrat des Tiotropiumbromids, welches durch die Wahl spezifischer Reaktionsbedingungen in kristalliner Form erhalten werden kann, eine wasserfreie Kristallmodifikation des Tiotropiumbromids erhalten werden kann, die den eingangs genannten hohen Anforderungen genügt und somit die der vorliegenden Erfindung zugrunde liegende Aufgabe löst. Dementsprechend betrifft die vorliegende Erfindung dieses kristalline, wasserfreie Tiotropiumbromid. Eine im Rahmen der vorliegenden Erfindung erfolgende Bezugnahme auf die Bezeichnung Tiotropiumbromid-anhydrat ist als Bezugnahme auf das erfindungsgemäße kristalline Tiotropiumbromid in wasserfreier Form anzusehen, welches durch eine mittels Röntgenstrukturanalyse bestimmte monoklinische Elementarzelle mit den Parametern a = 10.4336(2)Å, b = 11.3297(3)Å, c = 17.6332(4) Å, α = 90°, β = 105.158(2)° und γ = 90° (Zellvolumen=2011.89(8) Å³) gekennzeichnet ist.

Ein weiterer Aspekt der vorliegenden Erfindung betrifft ein Herstellverfahren der kristallinen Form des wasserfreien Tiotropiumbromids. Dieses Herstellverfahren ist dadurch gekennzeichnet, daß Tiotropiumbromid, welches beispielsweise nach der in der EP 418 716 A1 offenbarten Herstellungsvorschrift erhalten worden ist, in Wasser aufgenommen wird, die erhaltene Mischung erwärmt wird und abschließend das Hydrat des Tiotropiumbromids unter langsamem Abkühlen kristallisiert werden. Aus dem so erhaltenen kristallinen Tiotropiumbromid-monohydrat kann dann durch Trocknung, wasserfreies, kristallines Tiotropiumbromid erhalten werden.

Die vorliegende Erfindung betrifft ferner kristallines, wasserfreies Tiotropiumbromid, welches durch vorstehende Vorgehensweise erhältlich ist.

Ein Aspekt der vorliegenden Erfindung betrifft ein Herstellverfahren von kristallinem, wasserfreien Tiotropiumbromid ausgehend von kristallinem Tiotropiumbromid-monohydrat, welches nachfolgend detaillierter beschrieben wird.
Zur Herstellung des kristallinen Monohydrats ist es erforderlich, Tiotropiumbromid, welches beispielsweise nach der in der EP 418 716 A1 offenbarten Herstellungsvorschrift erhalten worden ist, in Wasser aufzunehmen, zu Erwärmen, eine Reinigung mit Aktivkohle durchzuführen und nach Abtrennen der Aktivkohle unter langsamem Abkühlen das Tiotropiumbromid-monohydrat langsam zu kristallisieren. Aus diesem Kristallisat wird durch vorsichtiges Erwärmen bei mehr als 50°C, vorzugsweise bei 60-100°C besonders bevorzugt bei 70-100°C unter vermindertem Druck, vorzugsweise im Hochvakuum über einen Zeitraum von 15 Minuten bis 24 Stunden, vorzugsweise 20 Minuten bis 12 Stunden die wasserfreie Form erhalten.

Erfindungsgemäß bevorzugt wird wie nachfolgend beschrieben vorgegangen.
In einem geeignet dimensionierten Reaktionsgefäß wird das Lösemittel mit Tiotropiumbromid, welches beispielsweise nach der in der EP 418 716 A1 offenbarten Herstellungsvorschrift erhalten worden ist, gemischt.
Pro Mol eingesetztes Tiotropiumbromid werden 0,4 bis 1,5 kg, bevorzugt 0,6 bis 1 kg, besonders bevorzugt ca. 0,8 kg Wasser als Lösemittel verwendet.

Die erhaltene Mischung wird unter Rühren erwärmt, vorzugsweise auf mehr als 50°C, besonders bevorzugt auf mehr als 60°C. Die maximal wählbare Temperatur bestimmt sich durch den Siedepunkt des verwendeten Lösemittels Wasser. Vorzugsweise wird die Mischung auf einen Bereich von 80-90°C erhitzt.

In diese Lösung wird Aktivkohle, trocken oder wasserfeucht, eingebracht. Bevorzugt werden pro Mol eingesetztes Tiotropiumbromid 10 bis 50 g, besonders bevorzugt 15 bis 35 g, höchst bevorzugt etwa 25 g Aktivkohle eingesetzt. Gegebenenfalls wird die Aktivkohle vor Einbringen in die Tiotropiumbromid-haltige Lösung in Wasser aufgeschlämmt. Pro Mol eingesetztes Tiotropiumbromid werden zum Aufschlämmen der Aktivkohle 70 bis 200 g, bevorzugt 100 bis 160 g, besonders bevorzugt ca. 135 g Wasser verwendet. Wird die Aktivkohle vor Einbringen in die Tiotropiumbromid-haltige Lösung zuvor in Wasser aufgeschlämmt, empfiehlt es sich, mit der gleichen Menge Wasser nachzuspülen.
Bei konstanter Temperatur wird nach erfolgter Aktivkohlezugabe zwischen 5 bis 60 Minuten, bevorzugt zwischen 10 und 30 Minuten, besonders bevorzugt etwa 15 Minuten weitergerührt und die erhaltene Mischung filtriert, um die Aktivkohle zu entfernen. Der Filter wird anschließend mit Wasser nachgespült. Hierfür werden pro Mol eingesetztes Tiotropiumbromid 140 bis 400 g, bevorzugt 200 bis 320 g, höchst bevorzugt ca. 270 g Wasser verwendet.
Das Filtrat wird anschließend langsam abgekühlt, vorzugsweise auf eine Temperatur von 20-25°C. Die Abkühlung wird vorzugsweise mit einer Abkühlrate von 1 bis 10°C pro 10 bis 30 Minuten, bevorzugt von 2 bis 8°C pro 10 bis 30 Minuten, besonders bevorzugt von 3 bis 5°C pro 10 bis 20 Minuten, höchst bevorzugt von 3 bis 5°C pro ca. 20 Minuten durchgeführt. Gegebenenfalls kann sich nach dem Abkühlen auf 20 bis 25°C eine weitere Abkühlung auf unter 20°C, besonders bevorzugt auf 10 bis 15°C anschließen.
Nach erfolgter Abkühlung wird zwischen 20 Minuten und 3 Stunden, vorzugsweise zwischen 40 Minuten und 2 Stunden, besonders bevorzugt etwa eine Stunde zur Vervollständigung der Kristallisation nachgerührt.
Die entstandenen Kristalle werden abschließend durch Filtrieren oder Absaugen des Lösemittels isoliert. Sollte es erforderlich sein, die erhaltenen Kristalle einem weiteren Waschschritt zu unterwerfen, empfiehlt es sich als Waschlösemittel Wasser oder Aceton zu verwenden. Pro Mol eingesetztes Tiotropiumbromid können zum Waschen der erhaltenen Tiotropiumbromid-monohydrat-Kristalle 0,1 bis 1,0 L, bevorzugt 0,2 bis 0,5 L, besonders bevorzugt etwa 0,3 L Lösemittel Verwendung finden. Gegebenenfalls kann der Waschritt wiederholt durchgeführt werden.
Das erhaltene Produkt wird im Vakuum oder mittels erwärmter Umluft bis zum Erreichen eines Wassergehalts von 2,5 - 4,0 % getrocknet.
Aus dem so erhaltenen kristallinen Tiotropiumbromid-monohydrat wird durch sorgfältiges Trocknen bei mehr als 50°C, vorzugsweise bei 60-100°C, besonders bevorzugt bei 70-100°C, unter vermindertem Druck, vorzugsweise im Hochvakuum über einen Zeitraum von 15 Minuten bis 24 Stunden, vorzugsweise 20 Minuten bis 12 Stunden, besonders bevorzugt 30 Minuten bis 6 Stunden die wasserfreie Form erhalten. Besonders bevorzugt wird unter vermindertem Druck ein Wert von bis zu 5 x 10⁻² bar, vorzugsweise 1 x 10⁻² bar, besonders bevorzugt 5 x 10⁻³ bar verstanden. Besonders bevorzugt wird vorstehend genannte Entwässerung zum Anhydrat bei etwa 1 x 10⁻³ bar oder weniger durchgeführt.

Alternativ zum vorstehend genannten Trocknungsschritt bei erhöhter Temperatur unter vermindertem Druck kann auch durch Lagerung des kristallinen Tiotropiumbromidmonohydrats über einem geeigneten Trocknungsmittel, vorzugsweise über getrocknetem Kieselgel bei Raumtemperatur über einen Zeitraum von 12 bis 96 Stunden, vorzugsweise 18 bis 72 Stunden, besonders bevorzugt wenigstens 24 Stunden die wasserfreie Form hergestellt werden. Die so erhaltene wasserfreie Form sollte je nach Partikelgröße mehr oder weniger trocken gelagert werden, um den wasserfreien Zustand zu konservieren. Im Falle von grobkristallinem wasserfreien Tiotropiumbromid, welches beispielsweise wie obenstehend beschrieben erzeugt werden kann, reicht eine Lagerung bei < 75 % r.F. (Raumfeuchtigkeit) aus, um den wasserfreien Zustand zu erhalten. Im mikronisierten Zustand, einem Material mit stark vergrößerter Oberfläche, kann die Wasseraufnahme gegebenenfalls bereits bei niedrigeren Luftfeuchten erfolgen. Um im mikronisierten Zustand die wasserfreie Form zu erhalten, empfiehlt es sich daher, die wasserfreie From des Tiotropiumbromids über getrocknetem Kieselgel zu lagern, bis eine Weiterverarbeitung zum gewünschten Inhaltationspulver erfolgt, welches neben Tiotropiumbromid geeignete Hilfsstoffe (beispielsweise Lactose) enthält.

Ein Aspekt der vorliegenden Erfindung betrifft kristallines, wasserfreies Tiotropiumbromid, welches gemäß vorstehend beschriebener Vorgehensweise erhältlich ist. Ferner betrifft die vorliegende Erfindung die Verwendung von kristallinem Tiotropiumbromid-monohydrat zur Herstellung von kristallinem Tiotropiumbromid in wasserfreier Form.

### Charakterisierung des kristallinen Tiotropiumbromid-monohydrats

Das gemäß vorstehend beschriebener Vorgehensweise erhältliche Tiotropiumbromid-monohydrat, welches als Ausgangsmaterial zu Herstellung des erfindungsgemäßen kristallinen, wasserfreien Tiotropiumbromids dient, wurde einer Untersuchung mittels DSC (Differential Scanning Calorimetry) unterworfen. Das DSC- diagramm weist zwei charakteristische Signale auf. Das erste, relativ breite, endotherme Signal zwischen 50-120°C ist auf die Entwässerung des Tiotropiumbomid-monohydrats zur wasserfreien Form zurückzuführen. Das zweite, relativ scharfe, endotherme Maximum bei 230 ± 5°C ist dem Schmelzen der Substanz unter Zersetzung zuzuordnen. Diese Daten wurden mittels eines Mettler DSC 821 erhalten und mit dem Mettler Software-Paket STAR ausgewertet. Die Daten wurden bei einer Heizrate von 10 K/min erhoben.
Da Tiotropiumbromid-monohydrat unter Zersetzung schmilzt (= inkongruenter Schmelzvorgang), hängt der beobachtete Schmelzpunkt sehr von der Heizrate ab. Bei geringeren Heizraten wird der Schmelz-/Zersetzungsvorgang bei deutlich niedrigeren Temperaturen beobachtet, so zum Beispiel mit einer Heizrate von 3 K/min bei 220 ± 5 °C. Es kann außerdem vorkommen, daß der Schmelzpeak aufgespalten vorliegt. Die Aufspaltung tritt umso stärker auf, je geringer die Heizrate im DSC-Experiment ist.

Das gemäß vorstehend beschriebener Vorgehensweise erhältliche Tiotropiumbromid-monohydrat, welches als Ausgangsmaterial zu Herstellung des erfindungsgemäßen kristallinen, wasserfreien tiotropiumbromids dient, wurde mittels IR-Spektroskopie charakterisiert. Die Daten wurden mittels eines Nicolet FTIR Spektrometers erhoben und mit dem Nicolet Softwarepaket OMNIC, version 3.1 ausgewertet. Die Messung wurde mit 2,5µmol Tiotropiumbromid-monohydrat in 300 mg KBr durchgeführt. Tabelle 1 faßt einige der wesentlichen Banden des IR-Spektrums zusammen.

**Tabelle 1: Zuordnung von spezifischen Banden**

| Wellenzahl (cm⁻¹) | Zuordnung | Schwingungstyp |
|---|---|---|
| 3570,3410 | O-H | Streckschwingung |
| 3105 | Aryl C-H | Streckschwingung |
| 1730 | C=O | Streckschwingung |
| 1260 | Epoxid C-O | Streckschwingung |
| 1035 | Ester C-OC | Streckschwingung |
| 720 | Thiophen | Ringschwingung |

Das gemäß vorstehend beschriebener Vorgehensweise erhältliche Tiotropiumbromid-monohydrat, welches als Ausgangsmaterial zu Herstellung des erfindungsgemäßen kristallinen, wasserfreien Tiotropiumbromids dient, wurde mittels Röntgenstrukturanalyse charakterisiert. Die Röntgenbeugungsintensitätsmessungen wurden auf einem AFC7R- 4-Kreisdiffraktometer (Rigaku) unter Verwendung von monochromatisierter Kupfer Kα-Strahlung durchgeführt. Die Strukturlösung und Verfeinerung der Kristallstruktur erfolgte mittels Direkter Methoden (Programm SHELXS86) und FMLQ-Vefeinerung (Programm TeXsan). Experimentelle Details zur Kristallstruktur, Strukturlösung und -verfeinerung sind in Tabelle 2 zusammengefaßt.

**Tabelle 2: Experimentelle Daten zur Kristallstrukturanalyse von Tiotropiumbromid-monohydrat.**

| A. Kristalldaten | |
|---|---|
| Empirische Formel | [C₁₉H₂₂NO₄S₂] Br · H₂O |
| Formelgewicht | 472.43 + 18.00 |
| Kristallfarbe, -gestalt | farblos, prismatisch |
| Kristallabmessungen | 0.2 x 0.3 x 0.3 mm |
| Kristallsystem | monoklin |
| Gittertyp | primitv |
| Raumgruppe | P 2₁/n |
| Gitterkonstanten | a = 18.0774 Å, |
| | b=11.9711Å |
| | c = 9.9321 Å |
| | β = 102.691° |
| | V = 2096.96 Å³ |
| Formeleinheiten pro Elementarzelle | 4 |

| B. Intensitätsmessungen | |
|---|---|
| Diffraktometer | Rigaku AFC7R |
| Röntgengenerator | Rigaku RU200 |
| Wellenlänge | λ = 1.54178 Å (monochromatisierte Kupfer Kα-Strahlung) |
| Strom, Spannung | 50kV, 100mA |
| Take-off Winkel | 6° |
| Kristallmontage | wasserdampfgesättigte Kapillare |
| Kristall-Detektor-Abstand | 235mm |
| Detektor Öffnung | 3.0 mm vertikal und horizontal |
| Temperatur | 18°C |
| Bestimmung der Gitterkonstanten | 25 Reflexe (50.8° < 2Θ < 56.2°) |
| Scan Typ | Θ - 2Θ |
| 2Θmax | 120° |
| Messungen | 5193 |
| Unabhängige Reflexe | 3281 (Rint=0.051) |
| Korrrekturen | Lorentz-Polarisation |
| | Absorption |
| | (Transmissionsfaktoren 0.56 -1.00) |
| | Kristall-decay 10.47% Abfall |

| C. Verfeinerung | |
|---|---|
| Reflexe (I > 3σl) | 1978 |
| Variable | 254 |
| Verhältnis Reflexe/Parameter | 7.8 |
| R-Werte: R, Rw | 0.062, 0.066 |

Die durchgeführte Röntgenstrukturanyluse ergab, daß kristallines Toptropiumbromidmonohydrat eine einfache monoklinische Zelle mit folgenden Dimensionen aufweist: a = 18.0774 Å, b = 11.9711 Å, c = 9.9321 Å, β = 102.691 °, V = 2096.96 Å³. Durch die vorstehende Röntgenstrukturanalyse wurden die in Tabelle 3 beschriebenen Atomkoordinaten bestimmt:

**Tabelle 3: Koordinaten**

| Atom | x | y | z | u (eq) |
|---|---|---|---|---|
| Br(1) | 0.63938(7) | 0.0490(1) | 0.2651(1) | 0.0696(4) |
| S(1) | 0.2807(2) | 0.8774(3) | 0.1219(3) | 0.086(1) |
| S(2) | 0.4555(3) | 0.6370(4) | 0.4214(5) | 0.141(2) |
| O(1) | 0.2185(4) | 0.7372(6) | 0.4365(8) | 0.079(3) |
| O(2) | 0.3162(4) | 0.6363(8) | 0.5349(9) | 0.106(3) |
| O(3) | 0.3188(4) | 0.9012(5) | 0.4097(6) | 0.058(2) |
| O(4) | 0.0416(4) | 0.9429(6) | 0.3390(8) | 0.085(3) |
| O(5) | 0.8185(5) | 0.0004(8) | 0.2629(9) | 0.106(3) |
| N(1) | 0.0111(4) | 0.7607(6) | 0.4752(7) | 0.052(2) |
| C(1) | 0.2895(5) | 0.7107(9) | 0.4632(9) | 0.048(3) |
| C(2) | 0.3330(5) | 0.7876(8) | 0.3826(8) | 0.048(3) |
| C(3) | 0.3004(5) | 0.7672(8) | 0.2296(8) | 0.046(3) |
| C(4) | 0.4173(5) | 0.7650(8) | 0.4148(8) | 0.052(3) |
| C(5) | 0.1635(5) | 0.6746(9) | 0.497(1) | 0.062(3) |
| C(6) | 0.1435(5) | 0.7488(9) | 0.6085(9) | 0.057(3) |
| C(7) | 0.0989(6) | 0.6415(8) | 0.378(1) | 0.059(3) |
| C(8) | 0.0382(5) | 0.7325(9) | 0.3439(9) | 0.056(3) |
| C(9) | 0.0761(6) | 0.840(1) | 0.315(1) | 0.064(3) |
| C(10) | 0.1014(6) | 0.8974(8) | 0.443(1) | 0.060(3) |
| C(11) | 0.0785(5) | 0.8286(8) | 0.5540(9) | 0.053(3) |
| C(12) | -0.0632(6) | 0.826(1) | 0.444(1) | 0.086(4) |
| C(13) | -0.0063(6) | 0.6595(9) | 0.554(1) | 0.062(3) |
| C(14) | 0.4747(4) | 0.8652(9) | 0.430(1) | 0.030(2) |
| C(15) | 0.2839(5) | 0.6644(9) | 0.1629(9) | 0.055(3) |
| C(16) | 0.528(2) | 0.818(2) | 0.445(2) | 0.22(1) |
| C(17) | 0.5445(5) | 0.702(2) | 0.441(1) | 0.144(6) |
| C(18) | 0.2552(6) | 0.684(1) | 0.019(1) | 0.079(4) |
| C(19) | 0.2507(6) | 0.792(1) | -0.016(1) | 0.080(4) |
| H(1) | -0.0767 | 0.8453 | 0.5286 | 0.102 |
| H(2) | -0.0572 | 0.8919 | 0.3949 | 0.102 |
| H(3) | -0.1021 | 0.7810 | 0.3906 | 0.102 |
| H(4) | -0.0210 | 0.6826 | 0.6359 | 0.073 |
| H(5) | -0.0463 | 0.6178 | 0.4982 | 0.073 |
| H(6) | 0.0377 | 0.6134 | 0.5781 | 0.073 |
| H(7) | 0.1300 | 0.7026 | 0.6770 | 0.069 |
| H(8) | 0.1873 | 0.7915 | 0.6490 | 0.069 |
| H(9) | 0.1190 | 0.6284 | 0.2985 | 0.069 |
| H(10) | 0.0762 | 0.5750 | 0.4016 | 0.069 |
| H(11) | 0.1873 | 0.6082 | 0.5393 | 0.073 |
| H(12) | -0.0025 | 0.7116 | 0.2699 | 0.066 |
| H(13) | 0.1084 | 0.8383 | 0.2506 | 0.075 |
| H(14) | 0.1498 | 0.9329 | 0.4626 | 0.071 |
| H(15) | 0.0658 | 0.8734 | 0.6250 | 0.063 |
| H(16) | 0.2906 | 0.5927 | 0.2065 | 0.065 |
| H(17) | 0.2406 | 0.6258 | -0.0469 | 0.094 |
| H(18) | 0.2328 | 0.8191 | -0.1075 | 0.097 |
| H(19) | 0.4649 | 0.9443 | 0.4254 | 0.037 |
| H(20) | 0.5729 | 0.8656 | 0.4660 | 0.268 |
| H(21) | 0.5930 | 0.6651 | 0.4477 | 0.165 |
| H(22) | 0.8192 | -0.0610 | 0.1619 | 0.084 |
| H(23) | 0.7603 | 0.0105 | 0.2412 | 0.084 |

| | | | | |
|---|---|---|---|---|
| x, y, z: fraktionelle Koordinaten; | | | | |
| U(eq) mittlere quadratische Amplitude atomarer Bewegung im Kristall; | | | | |

### Charakterisierung des kristallinen, wasserfreien Tiotropiumbromids

Wie vorstehend beschrieben wird ausgehend von kristallinem Tiotropiumbromid-monohydrat das erfindungsgemäße, kristalline wasserfreie Tiotropiumbromid zugänglich.
Die kristalline Struktur des wasserfreien Tiotropiumbromids wurde aus hochauflösenden Röntgenpulverdaten (Synchrotronstrahlung) durch einen Realraum-Ansatz über ein sog. "simuliertes Temperverfahren" (simulated annealing) bestimmt. Eine abschließende Rietveld-Analyse wurde zur Verfeinerung der Strukturparameter durchgeführt. Tabelle 4 faßt die für kristallines, wasserfreies Tiotropiumbromid erhaltenen experimentellen Daten zusammen.

**Tabelle 4: Experimentelle Daten zur Kristallstrukturanalyse von Tiotropiumbromid (wasserfrei)**

| | |
|---|---|
| Formel | C₁₉H₂₂NO₄S₂Br |
| Temperatur [°C] | 25 |
| Molekulargewicht [g/mol] | 472.4 |
| Raumgruppe | *P*2₁/*c* |
| *a* [Å] | 10.4336(2) |
| *b* [Å] | 11.3297(3) |
| *c* [Å] | 17.6332(4) |
| β [°] | 105.158(2) |
| V [Å3] | 2011.89(8) |
| Z | 4 |
| berechnete Dichte [g cm⁻³] | 1.56 |
| 20 (Bereich) [°] | 2.0-20 |
| Intervall [°2Θ] | 0.003 |
| Zählzeit / Schritt [sec] | 3 |
| Wellenlänge [Å] | 0.7000 |

Dementsprechend betrifft die vorliegende Erfindung kristallines, wasserfreies Tiotropiumbromid, welches durch die Elementarzelle
a = 10.4336(2)Å,
b = 11.3297(3)Å,
c = 17.6332(4) Å und
α = 90°,
β = 105.158(2)° und
γ = 90° (Zellvolumen = 2011.89(8) Å³).
gekennzeichnet ist.

Die Kristallstruktur der wasserfreien Form des Tiotropiumbromids kann als Schichtstruktur beschrieben werden. Zwischen den aus Tiotropium gebildeten Schichten sind die Bromidionen lokalisiert.

Zur Strukturaufklärung des kristallinen, wasserfreien Tiotropiumbromids wurde ein hochaufgelöstes Röntgenpulverdiagramm bei Raumtemperatur an der Nationalen Synchrotronquelle (Brookhaven National Laboratory, USA) an der Meßstation X3B1 (λ = 0.700 Å) aufgenommen. Für dieses Experiment wurde eine Probe von kristallinem Tiotropiumbromid-monohydrat in eine Quarzglaskapillare mit 0,7 mm Durchmesser eingebracht. In einem Ofen wurde das Wasser durch Aufheizen unter vermindertem Druck auf 80°C entfernt

Die Sturkturlösung erfolgte durch ein sog. "simuliertes Temperverfahren". Hierzu wurde das Progammpacket DASH vom Cambridge Crystallographic Data Center (CCDC, Cambridge, United Kingdom) verwendet.

Tabelle 5 faßt die für kristallines, wasserfreies Tiotropiumbromid ermittelten Atomkoordinaten zusammen.

**Tabelle 5: Koordinaten**

| Atom | x | y | z | Uᵢₛₒ |
|---|---|---|---|---|
| S1 | 1.0951(8) | 0.3648(8) | 0.8189(5) | 0.075(9) |
| S1 | 0.9143(9) | 0.1374(8) | 0.9856(5) | 0.075(9) |
| O | 0.6852(13) | 0.2339(6) | 0.7369(6) | 0.075(9) |
| O1 | 0.7389(15) | 0.0898(9) | 0.8234(6) | 0.075(9) |
| O2 | 0.8211 (10) | 0.3897(17) | 0.8277(7) | 0.075(9) |
| O3 | 0.4975(17) | 0.4816(9) | 0.6011 (7) | 0.075(9) |
| N | 0.4025(10) | 0.2781 (8) | 0.5511 (5) | 0.075(9) |
| C | 0.7509(8) | 0.1885(6) | 0.8038(5) | 0.075(9) |
| C1 | 0.8593(7) | 0.2788(5) | 0.8495(4) | 0.075(9) |
| C2 | 0.9924(9) | 0.2533(6) | 0.8225(6) | 0.075(9) |
| C3 | 0.8884(9) | 0.2664(7) | 0.9382(4) | 0.075(9) |
| C4 | 0.5848(12) | 0.1596(8) | 0.6753(8) | 0.075(9) |
| C5 | 0.4544(13) | 0.1929(14) | 0.6809(8) | 0.075(9) |
| C6 | 0.6156(13) | 0.1810(13) | 0.5973(9) | 0.075(9) |
| C7 | 0.5493(11) | 0.2881(11) | 0.5578(6) | 0.075(9) |
| C8 | 0.5869(12) | 0.3832(11) | 0.6092(7) | 0.075(9) |
| C9 | 0.4947(13) | 0.3902(10) | 0.6575(6) | 0.075(9) |
| C10 | 0.4004(10) | 0.2998(11) | 0.6332(6) | 0.075(9) |
| C11 | 0.3220(13) | 0.3670(13) | 0.4935(6) | 0.075(9) |
| C12 | 0.3450(19) | 0.1643(26) | 0.5211(11) | 0.075(9) |
| C13 | 0.9184(16) | 0.3808(9) | 0.9920(6) | 0.075(9) |
| C14 | 1.0313(16) | 0.1552(15) | 0.8011(15) | 0.075(9) |
| C15 | 0.9515(17) | 0.3374(10) | 0.0501(6) | 0.075(9) |
| C16 | 0.9756(18) | 0.2190(11) | 1.0742(5) | 0.075(9) |
| C17 | 1.1483(22) | 0.1762(18) | 0.7718(24) | 0.075(9) |
| C18 | 1.1860(16) | 0.2800(15) | 0.7768(19) | 0.075(9) |
| BR | 0.4597(4) | 0.8200(15) | 0.61902(25) | 0.042(9) |

In vorstehender Tabelle bezeichnen die Werte "Uᵢₛₒ" die isotropen Temperaturfaktoren. Beispeilsweise in der Einkristallröntgenstrukturanalyse entspricht dies den u(eq)-Werten.

Tabelle 6 faßt die Reflexe (h,k,l-Indizes) des für kristallines, wasserfreies Tiotropiumbromid erhaltenen Pulverdiagramms zusammen.

**Tabelle 6: Experimentelle Daten zur Kristallstrukturanalyse von wasserfreiem Tiotropiumbromid**

| Nr. | h | k | l | 2Θ obs. | 2Θ calc. | 2Θ obs. - 2Θ calc |
|---|---|---|---|---|---|---|
| 1 | 1 | 0 | 0 | 8.762 | 8.769 | -0.007 |
| 2 | 0 | 1 | 1 | 9.368 | 9.369 | -0.001 |
| 3 | -1 | 0 | 2 | 11.730 | 11.725 | 0.005 |
| 4 | 0 | 1 | 2 | 12.997 | 13.004 | -0.007 |
| 5 | -1 | 1 | 2 | 14.085 | 14.094 | -0.009 |
| 6 | 1 | 0 | 2 | 15.271 | 15.275 | -0.004 |
| 7 | 0 | 0 | 3 | 15.620 | 15.616 | 0.004 |
| 8 | 0 | 2 | 1 | 16.475 | 16.475 | 0.0 |
| 9 | 1 | 1 | 2 | 17.165 | 17.170 | -0.005 |
| 10 | 2 | 0 | 0 | 17.588 | 17.591 | -0.003 |
| 11 | -1 | 2 | 1 | 18.009 | 18.035 | -0.026 |
| 12 | 1 | 2 | 1 | 19.336 | 19.328 | 0.008 |
| 13 | -2 | 1 | 2 | 19.596 | 19.600 | -0.004 |
| 14 | -1 | 0 | 4 | 20.417 | 20.422 | -0.005 |
| 15 | 0 | 0 | 4 | 20.865 | 20.872 | -0.007 |
| 16 | 2 | 1 | 1 | 21.150 | 21.145 | 0.005 |
| 17 | -2 | 1 | 3 | 21.759 | 21.754 | 0.005 |
| 18 | 0 | 2 | 3 | 22.167 | 22.160 | 0.007 |
| 19 | -1 | 2 | 3 | 22.289 | 22.288 | 0.001 |
| 20 | 2 | 0 | 2 | 22.735 | 22.724 | 0.011 |
| 21 | -2 | 2 | 1 | 23.163 | 23.159 | 0.004 |
| 22 | -2 | 0 | 4 | 23.567 | 23.575 | -0.008 |
| 23 | 2 | 1 | 2 | 24.081 | 24.058 | 0.023 |
| 24 | 1 | 0 | 4 | 24.746 | 24.739 | 0.007 |
| 25 | -1 | 3 | 1 | 25.220 | 25.221 | -0.001 |
| 26 | 1 | 2 | 3 | 25.359 | 25.365 | -0.006 |
| 27 | 0 | 3 | 2 | 25.790 | 25.783 | 0.007 |
| 28 | 1 | 1 | 4 | 25.978 | 25.975 | 0.003 |
| 29 | 0 | 2 | 4 | 26.183 | 26.179 | 0.004 |
| 30 | -1 | 3 | 2 | 26.383 | 26.365 | 0.018 |
| 31 | -1 | 1 | 5 | 26.555 | 26.541 | 0.014 |
| 32 | -3 | 1 | 2 | 27.024 | 27.021 | 0.003 |
| 33 | 3 | 1 | 0 | 27.688 | 27.680 | 0.008 |
| 34 | -3 | 1 | 3 | 28.221 | 28.215 | 0.006 |
| 35 | 3 | 0 | 1 | 28.377 | 28.376 | 0.001 |
| 36 | -3 | 0 | 4 | 29.246 | 29.243 | 0.003 |
| 37 | 3 | 1 | 1 | 29.459 | 29.471 | -0.012 |
| 38 | -1 | 2 | 5 | 29.906 | 29.900 | 0.006 |
| 39 | -3 | 2 | 1 | 30.171 | 30.165 | 0.006 |
| 40 | 0 | 2 | 5 | 30.626 | 30.626 | 0.0 |
| 41 | 1 | 1 | 5 | 30.871 | 30.856 | 0.015 |
| 42 | 0 | 0 | 6 | 31.504 | 31.532 | -0.028 |
| 43 | 2 | 1 | 4 | 31.826 | 31.847 | -0.021 |
| 44 | -2 | 1 | 6 | 32.888 | 32.888 | 0.0 |
| 45 | 1 | 4 | 1 | 33.605 | 33.615 | -0.010 |
| 46 | 3 | 0 | 3 | 34.379 | 34.377 | 0.002 |
| 47 | 1 | 0 | 6 | 35.021 | 35.018 | 0.003 |
| 48 | -4 | 1 | 1 | 35.513 | 35.503 | 0.01 |
| 49 | 1 | 1 | 6 | 35.934 | 35.930 | 0.004 |
| 50 | -1 | 1 | 7 | 36.544 | 36.543 | 0.001 |
| 51 | -4 | 1 | 4 | 37.257 | 37.255 | 0.002 |
| 52 | -4 | 2 | 2 | 37.933 | 37.952 | -0.019 |
| 53 | 4 | 1 | 1 | 38.258 | 38.264 | -0.006 |

Ein weiterer Aspekt der vorliegenden Erfindung betrifft aufgrund der pharmazeutischen Wirksamkeit der erfindungsgemäßen wasserfreien Form die Verwendung von kristallinem, wasserfreiem Tiotropiumbromid als Arzneimittel.
Zur Herstellung eines inhalativ applizierbaren Arzneimittels, insbesondere eines Inhalationspulvers, welches das durch die vorliegende Erfindung beschriebene wasserfreie, kristalline Tiotropiumbromid enthält, kann nach im Stand der Technik bekannten Verfahren vorgegangen werden. Diesbezüglich wird beispielsweise auf die Lehre der DE-A-179 22 07 verwiesen. Dementsprechend zielt ein weiterer Aspekt der vorliegenden Erfindung auf Inhaltionspulver gekennzeichnet durch einen Gehalt von kristallinem, wasserfreiem Tiotropiumbromid.

Aufgrund der hohen Wirksamkeit von Tiotropiumbromid enthalten die vorstehend genannten Inhalationspulver neben dem Wirkstoff vorzugsweise ferner physiologisch verträgliche Hilfsstoffe. Hierbei können beispielsweise die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

Die Hilfsstoffe weisen im Rahmen der erfindungsgemäßen Inhaltionspulver, die durch einen Gehalt an wasserfreiem, kristallinem Tiotropiumbromid gekennzeichnet sind, eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfststoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen.

Bevorzugte, das erfindungsgemäße Tiotropiumbromid-anhydrat enthaltende Inhalationspulver sind dadurch gekennzeichnet, daß der Hilfsstoff aus einem Gemisch von gröberem Hilfsstoff mit einer mittleren Teilchengröße von 17 bis 50µm, besonders bevorzugt von 20 bis 30 µm und feinerem Hilfststoff mit einer mittleren Teilchengröße von 2 bis 8µm, besonders bevorzugt von 3 bis 7µm besteht. Dabei wird unter der mittleren Teilchengröße im hier verwendeten Sinne der 50%-Wert aus der Volumenverteilung gemessen mit einem Laserdiffraktometer nach der Trockendispersionsmethode verstanden. Bevorzugt sind Inhalationspulver bei denen der Anteil von feinerem Hilfsstoff an der Gesamthilfsstoffmenge 3 bis 15%, besonders bevorzugt 5 bis 10% beträgt.

Ein mögliches Verfahren zur Herstellung dieser erfindungsgemäß bevorzugten Inhaltionspulver ist nachstehend detaillierter erörtert.
Nach Einwaage der Ausgangsmaterialien erfolgt zunächst die Fertigung der Hilfsstoffmischung aus den definierten Fraktionen des gröberen Hilfsstoffs und des feineren Hilfsstoffs. Anschließend erfolgt die Herstellung der erfindungsgemäßen Inhalationspulver aus der Hilfsstoffmischung und dem Wirkstoff. Soll das Inhalationspulver mittels Inhaletten in hierzu geeigneten Inhalatoren appliziert werden, schließt sich der Herstellung der Inhalationspulver die Fertigung der pulverhaltigen Kapseln an.

Die Herstellung der erfindungsgemäßen Inhaltionspulver erfolgt durch Mischen der gröberen Hilfsstoffanteile mit den feineren Hilfststoffanteilen und andschließendem Mischen der so erhaltenen Hilfsstoffgemische mit dem Wirkstoff.
Zur Herstellung der Hilfsstoffmischung werden die gröberen und feineren Hilfsstoffanteile in einen geeigneten Mischbehälter eingebracht. Die Zugabe der beiden Komponenten erfolgt vorzugsweise über einen Siebgranulator mit einer Maschenweite von 0,1 bis 2 mm, besonders bevorzugt 0,3 bis 1 mm, höchst bevorzugt 0,3 bis 0,6 mm. Vorzugsweise wird der gröbere Hilfsstoff vorgelegt und anschließend der feinere Hilfsstoffanteil in den Mischbehälter eingebracht. Bevorzugt erfolgt bei diesem Mischverfahren die Zugabe der beiden Komponenten portionsweise, wobei ein Teil des gröberen Hilfsstoffs zunächst vorgelegt und anschließend abwechselnd feinerer und gröberer Hilfsstoff zugegeben wird. Besonders bevorzugt ist bei der Herstellung der Hilfsstoffmischung das abwechselnde, schichtweise Einsieben der beiden Komponenten. Vorzugsweise erfolgt das Einsieben der beiden Komponenten abwechselnd in je 15 bis 45, besonders bevorzugt in je 20 bis 40 Schichten. Der Mischvorgang der beiden Hilfsstoffe kann bereits während der Zugabe der beiden Komponenten erfolgen. Vorzugsweise wird allerdings erst nach dem schichtweisen Einsieben der beiden Bestandteile gemischt.

Nach Herstellung der Hilfsstoffmischung werden diese und der Wirkstoff in einen geeigneten Mischbehälter eingebracht. Der verwendete Wirkstoff weist eine mittlere Teilchengröße von 0,5 bis 10µm, vorzugsweise von 1 bis 6µm, besonders bevorzugt von 2 bis 5µm auf. Die Zugabe der beiden Komponenten erfolgt vorzugsweise über einen Siebgranulator mit einer Maschenweite von 0,1 bis 2 mm, besonders bevorzugt 0,3 bis 1 mm, höchst bevorzugt 0,3 bis 0,6 mm. Vorzugsweise wird die Hilfsstoffmischung vorgelegt und anschließend der Wirkstoff in den Mischbehälter eingebracht. Bevorzugt erfolgt bei diesem Mischverfahren die Zugabe der beiden Komponenten portionsweise. Besonders bevorzugt ist bei der Herstellung der Hilfsstoffmischung das abwechselnde, schichtweise Einsieben der beiden Komponenten. Vorzugsweise erfolgt das Einsieben der beiden Komponenten abwechselnd in je 25 bis 65, besonders bevorzugt in je 30 bis 60 Schichten. Der Mischvorgang der Hilfsstoffmischung mit dem Wirkstoff kann bereits während der Zugabe der beiden Komponenten erfolgen. Vorzugsweise wird allerdings erst nach dem schichtweisen Einsieben der beiden Bestandteile gemischt.
Die so erhaltene Pulvermischung kann gegebenenfalls erneut ein- oder mehrfach über einen Siebgranulator gegeben und jeweils anschließend einem weiteren Mischvorgang unterworfen werden.

Die nach vorstehendem Verfahren erhältlichen Inhalationspulver enthalten vorzugsweise etwa 0.001 - 2 % Tiotropium im Gemisch mit einem physiologisch verträglichen Hilfsstoff. Bevorzugt sind Inhalationspulver, enthaltend 0,04 bis 0,8% Tiotropium im Gemisch mit einem physiologisch unbedenklichen Hilfsstoff, dadurch gekennzeichnet, daß der Hilfsstoff aus einem Gemisch von gröberem Hilfsstoff mit einer mittleren Teilchengröße von 15 bis 80µm und feinerem Hilfststoff mit einer mittleren Teilchengröße von 1 bis 9 µm besteht, wobei der Anteil von feinerem Hilfsstoff an der Gesamthilfsstoffmenge 1 bis 20% beträgt.
Erfindungsgemäß bevorzugt sind Inhalationspulver, die 0,08 bis 0,64%, besonders bevorzugt 0,16 bis 0,4% Tiotropium enthalten.

Insofern in die vorstehend genannten Inhalationspulver wasserfreies, kristallines Tiotropiumbromid eingesetzt wird, entahlten diese Pulvermischungen vorzugsweise 0,0012 - 2,41 % Tiotropiumbromid-anhydrat. Entsprechend bevorzugt sind Inhalationspulver, die zwischen 0,048 und 0,96% Tiotropiumbromid-anhydrat enthalten. Erfindungsgemäß von besonderem Interesse sind Inhalationspulver, die 0,096 bis 0,77%, besonders bevorzugt 0,19 bis 0,48% Tiotropiumbromid-anhydrat enthalten.

Bei den im Rahmen der vorliegenden Erfindung genannten prozentualen Angaben, handelt es sich stets um Gewichtsprozent.

Eine alternative, ebenfalls bevorzugte Ausführungsform zur Herstellung von Tiotropiumbromid-anhydrat enthaltenden Inhaltionspulvern, kann ferner ausgehend von Inhalationspulvem erfolgen, die auf Basis des kristallinen Tiotropiumbromid-monohydrats formuliert wurden. Diese enthalten zwischen 0,0012 und 2,5%, vorzugsweise 0,05 bis 1 %, bevorzugt 0,1 bis 0,8%, besonders bevorzugt 0,2 bis 0,5% kristallines Tiotropiumbromid-monohydrat und sind vorzugsweise in Analogie zu dem vorstehend beschriebenen Verfahren zugänglich. Diese, kristallines Tiotropiumbromid-monohydrat enthaltenden Inhaltionspulver können zur Darstellung von Inhaltionspulvern, enthaltend das erfindungsgemäße Tiotropiumbromid-anhydrat, entweder vor Befüllung der entsprechenden Inhalationskapseln oder bevorzugt nach Abfüllung in die entsprechenden Inhalationskapseln bei mehr als 60°C, vorzugsweise bei 65-100°C, besonders bevorzugt bei 70-100°C, unter vermindertem Druck, vorzugsweise im Hochvakuum über einen Zeitraum von 15 Minuten bis 24 Stunden, vorzugsweise von 20 Minuten bis 12 Stunden, besonders bevorzugt von 30 Minuten bis 6 Stunden getrocknet werden. Besonders bevorzugt wird unter vermindertem Druck ein Wert von bis zu 5 x 10⁻² bar, vorzugsweise 1 x 10⁻² bar, besonders bevorzugt 5 x 10⁻³ bar verstanden.

Besonders beverzugt wird vorstehend genannte Entwässerung zum Anhydrat bei etwa 1 x 10⁻³ bar oder weniger durchgeführt.

Aufgrund der anticholinergen Wirksamkeit von Tiotropiumbromid zielt ein weiterer Aspekt der vorliegenden Erfindung auf die Verwendung von kristallinem, wasserfreiem Tiotropiumbromid zur Herstellung eines Arzneimittels zur Behandlung von Erkrankungen, in denen die Applikation eines Anticholinergikums einen therapeutischen Nutzen entfalten kann. Bevorzugt ist die entsprechende Verwendung zur Herstellung eines Arzneimittels zur Behandlung von Asthma oder COPD.

Das nachfolgende Synthesebeispiel dient der Illustration eines exemplarisch durchgeführter Herstellungsverfahrens für wasserfreies, kristallines Tiotropiumbromid. Es ist lediglich als mögliche, exemplarisch dargestellte Vorgehensweise zu verstehen, ohne die Erfindung auf dessen Inhalt zu beschränken.

### Synthesebeispiel

### A) Herstellung von kristallinem Tiotropiumbromid-monohydrat:

In einem geeigneten Reaktionsgefäß werden in 25,7 kg Wasser 15,0 kg Tiotropiumbromid eingetragen. Die Mischung wird auf 80-90°C erhitzt und bei gleichbleibender Temperatur solange gerührt, bis eine klare Lösung entsteht. Aktivkohle (0,8 kg), wasserfeucht, wird in 4,4 kg Wasser aufgeschlämmt, diese Mischung in die Tiotropiumbromid-haltige Lösung eingetragen und mit 4,3 kg Wasser nachgespült. Die so erhaltene Mischung wird wenigstens 15 min bei 80-90°C gerührt und anschließend über einen beheizten Filter in einen auf 70°C Manteltemperatur vorgewärmten Apparat filtriert. Der Filter wird mit 8,6 kg Wasser nachgespült. Der Apparateinhalt wird mit 3-5°C pro 20 Minuten auf eine Temperatur von 20-25°C abgekühlt. Mit Kaltwasserkühlung wird der Apparat auf 10-15°C weiter abgekühlt und die Kristallisation durch mindestens einstündiges Nachrühren vervollständigt. Das Kristallisat wird über einen Nutschentrockner isoliert, der isolierte Kristallbrei mit 9 L kaltem Wasser (10-15°C) und kaltem Aceton (10-15°C) gewaschen. Die erhaltenen Kristalle werden bei 25°C über 2 Stunden im Stickstoffstrom getrocknet.
Ausbeute : 13,4 kg Tiotropiumbromid-monohydrat (86 % d. Th.)

### B) Herstellung von kristallinem, wasserfreiem Tiotropiumbromid:

Aus dem wie oben beschriebenen erhaltenen kristallinen Tiotropiumbromid-monohydrat wird durch sorgfältiges Trocknen bei 80 -100 °C unter vermindertem Druck, vorzugsweise im Hochvakuum (bei etwa 1 x 10⁻³ bar oder weniger) über einen Zeitraum von mindestens 30 Minuten die wasserfreie Form erzeugt. Alternativ zum Trocknungsschritt bei 80 - 100 °C im Vakuum kann auch durch Lagerung über getrocknetem Kieselgel bei Raumtemperatur über einen Zeitraum von mindestens 24 Stunden die wasserfreie Form hergestellt werden.

## Patentansprüche

1. Wasserfreies, kristallines Tiotropiumbromid, **gekennzeichnet durch** eine mittels Röntgenstrukturanalyse bestimmte monoklinische Elementarzelle mit den Parametern a = 10.4336(2)Å, b = 11.3297(3)Å, c = 17.6332(4) Å, α = 90°, β = 105.158(2)° und γ = 90° (Zellvolumen=2011.89(8) Å³).

2. Verfahren zur Herstellung von kristallinem, wasserfreiem Tiotropiumbromid gemäß Anspruch 1, **dadurch gekennzeichnet, daß** es durch sorgfältiges Trocknen bei mehr als 50°C, vorzugsweise bei 60-100°C, unter vermindertem Druck hergestellt wird.

3. Verfahren gemäß Anspruch 2, **dadurch gekennzeichnet, daß** die Trocknung über einen Zeitraum von 15 Minuten bis 24 Stunden erfolgt.

4. Verfahren zur Herstellung von kristallinem, wasserfreiem Tiotropiumbromid, gemäß Anspruch 1, **dadurch gekennzeichnet, daß** es durch Lagerung von kristallinen Tiotropiumbromidmonohydrat über einem geeigneten Trocknungsmittel über einen Zeitraum von 12 bis 96 Stunden hergestellt wird.

5. Kristallines, wasserfreies Tiotropiumbromid, erhältlich nach einem der Ansprüche 2, 3 oder 4.

6. Verwendung von kristallinem Tiotropiumbromid-monohydrat zur Herstellung von kristallinem, wasserfreiem Tiotropiumbromid.

7. Arzneimittel **gekennzeichnet durch** einen Gehalt an kristallinem, wasserfreiem Tiotropiumbromid nach einem der Ansprüche 1 oder 5.

8. Arzneimittel nach Anspruch 7, **dadurch gekennzeichnet, daß** es sich um ein Inhalationspulver handelt.

9. Inhalationspulver nach Anspruch 8, **dadurch gekennzeichnet, daß** es kristallines wasserfreies Tiotropiumbromid nach Anspruch 1 oder 5 im Gemisch mit einem physiologisch verträglichen Hilfsstoff enthält.

10. Inhalationspulver nach Anspruch 9, **dadurch gekennzeichnet, daß** der Hilfsstoff ausgewählt ist aus der Gruppe bestehend aus Glucose und Lactose.

11. Verwendung von kristallinem, wasserfreiem Tiotropiumbromid nach einem der Ansprüche 1 oder 5 zur Herstellung eines Arzneimittels zur Behandlung von Erkrankungen, in denen die Applikation eines Anticholinergikums einen therapeutischen Nutzen entfalten kann.

12. Verwendung nach Anspruch 11, **dadurch gekennzeichnet, daß** es sich bei den Erkrankungen um Asthma oder COPD handelt.

## Claims

1. Anhydrous crystalline tiotropium bromide, **characterised by** a monoclinic elementary cell with the parameters a = 10.4336(2)Å, b = 11.3297(3)Å, c = 17.6332(4) Å, α = 90°, β = 105.158(2)° and λ = 90° (cell volume = 2011.89(8) Å³) determined by X-ray structural analysis.

2. Process for preparing crystalline, anhydrous tiotropium bromide according to claim 1, **characterised in that** it is prepared by careful drying at more than 50°C, preferably at 60-100°C, under reduced pressure.

3. Process according to claim 2, **characterised in that** the drying is carried out over a period of from 15 minutes to 24 hours.

4. Process for preparing crystalline, anhydrous tiotropium bromide, according to claim 1, **characterised in that** it is prepared by storing crystalline tiotropium bromide monohydrate over a suitable drying agent for a period of 12 to 96 hours.

5. Crystalline anhydrous tiotropium bromide, obtainable according to one of claims 2, 3 or 4.

6. Use of crystalline tiotropium bromide monohydrate for preparing crystalline anhydrous tiotropium bromide.

7. Pharmaceutical composition, **characterised in that** it contains crystalline anhydrous tiotropium bromide according to one of claims 1 or 5.

8. Pharmaceutical composition according to claim 7, **characterised in that** it is an inhalable powder.

9. Inhalable powder according to claim 8, **characterised in that** it contains crystalline anhydrous tiotropium bromide according to claim 1 or 5 in admixture with a physiologically acceptable excipient.

10. Inhalable powder according to claim 9, **characterised in that** the excipient is selected from the group consisting of glucose and lactose.

11. Use of crystalline anhydrous tiotropium bromide according to one of claims 1 or 5 for preparing a pharmaceutical composition for the treatment of diseases in which the administration of an anticholinergic may have a therapeutic benefit.

12. Use according to claim 11, **characterised in that** the diseases are asthma or COPD.

## Revendications

1. Bromure de tiotropium cristallin anhydre **caractérisé par** une maille élémentaire monoclinique déterminée par analyse structurale aux rayons X ayant les paramètres : a = 10,4336(2) Å, b = 11,3297(3) Å, c = 17,6332(4) Å, α = 90°, β =105,158(2)° et γ = 90° (volume de maille = 2011,89(8) Å³).

2. Procédé de production de bromure de tiotropium anhydre cristallin selon la revendication 1 **caractérisé en ce qu'**il est produit par séchage soigneux à plus de 50°C, de préférence à 60-100°C, sous pression réduite.

3. Procédé selon la revendication 2 **caractérisé en ce que** le séchage a lieu pendant une durée de 15 minutes à 24 heures.

4. Procédé de production de bromure de tiotropium anhydre cristallin selon la revendication 1 **caractérisé en ce qu'**il est produit par stockage de bromure de tiotropium monohydraté cristallin au dessus d'un agent desséchant approprié pendant une durée de 12 à 96 heures.

5. Bromure de tiotropium anhydre cristallin pouvant être obtenu selon l'une des revendications 2, 3 ou 4.

6. Utilisation de bromure de tiotropium monohydraté cristallin pour la production de bromure de tiotropium anhydre cristallin.

7. Médicament **caractérisé par** une teneur en bromure de tiotropium anhydre cristallin selon l'une des revendications 1 ou 5.

8. Médicament selon la revendication 7 **caractérisé en ce qu'**il s'agit d'une poudre à inhaler.

9. Poudre à inhaler selon la revendication 8 **caractérisée en ce qu'**elle contient du bromure de tiotropium anhydre cristallin selon la revendication 1 ou 5 en mélange avec un adjuvant physiologiquement acceptable.

10. Poudre à inhaler selon la revendication 9 **caractérisée en ce que** l'adjuvant est choisi dans le groupe consistant en le glucose et le lactose.

11. Utilisation du bromure de tiotropium anhydre cristallin selon l'une des revendications 1 ou 5 pour la production d'un médicament pour le traitement des maladies dans lesquelles l'application d'un anticholinergique peut présenter une utilité thérapeutique.

12. Utilisation selon la revendication 11 **caractérisée en ce que** les maladies sont l'asthme ou la COPD.
